# EUROPÄISCHE PATENTANMELDUNG

(11) **EP 2 719 421 A1**
(43) Veröffentlichungstag der Anmeldung: **16.04.2014**
(21) Anmeldenummer: 13168215.5
(22) Anmeldetag: 17.05.2013
(51) Int. Cl.: A61N 1/05, A61B 5/00, A61B 5/04, A61B 5/042, A61B 5/0492, A61M 25/00

(54) **Implantierbare medizinische Schlauchanordnung**

(30) Priorität: 02.07.2012 US 201261666934 P
(71) Anmelder: BIOTRONIK SE & Co. KG, 12359 Berlin (DE)
(72) Erfinder: Weitzig, Pierre, 10249 Berlin (DE); Kolberg, Gernot, 12161 Berlin (DE)

(57) **Zusammenfassung**

Eine implantierbare medizinische Schlauchanordnung, umfasst eine auf Biege-, Zug- und radiale Drucklasten beanspruchbare Schlauchwand (1), die aus einem wendelförmig auf Block gewickelten Profilteil (2) gebildet ist, dessen in Wendel-Umlaufrichtung verlaufender Seitenrand (3) mit dem Seitenrand (4) des benachbarten Abschnitts des Profilteils (2) durch eine Eingriffsmimik (5) verbunden ist. Mit dem Profilteil (2) ist mindestens ein elektrisch leitfähiger, draht- oder seilartiger Einlagestrang (6) verbunden. Auch eine elektrisch leitfähige Ausrüstung des Profilteils (2) selbst ist möglich.

## Beschreibung

Die Erfindung betrifft eine implantierbare medizinische Schlauchanordnung, die eine auf Biege-, Zug- und radiale Drucklasten beanspruchbare Schlauchwand aufweist.

Derartige Schlauchanordnungen sind in erster Linie bei Elektrodenkathetern bekannt und im Einsatz, andere Anwendungsgebiete sind (teil-)implantierbare Schläuche ohne elektrische Funktionalität, wie beispielsweise Infusionsschläuche für externe oder implantierbare Infusionspumpen.

Im Folgenden soll die der Erfindung zugrundeliegende Problematik anhand des Beispiels eines Elektrodenkatheters erörtert werden. So weisen solche Elektrodenkatheter eine gewendelte Zuleitung als metallischer, draht- oder seilartiger Einlagestrang auf, der durch einen Elektrodenkörper aus einem Isolationsmaterial stabilisiert wird. Dieser Elektrodenkörper nimmt radiale Punkt- oder Linienlasten auf und verteilt sie in einem größeren Bereich der Wendel. Die Linienlast wird also im Wesentlichen in eine Flächenlast umgewandelt. Die Steifheit des Isolationsmaterials des Elektrodenkörpers reduziert die Biegespannung der Wendel bei auftretender Biegebeanspruchung. Ferner isoliert der Elektrodenkörper die Wendel mit zwei unterschiedlichen Zielrichtungen. So verhindert der Elektrodenkörper einerseits einen Kontakt zwischen der Wendel und eindringenden Körperflüssigkeiten, was zu Korrosionen an der Wendel führen würde. Zum anderen wird die Wendel natürlich auch zum Körper hin abgeschirmt, um eine elektrische Beaufschlagung des Körpers an nicht vorgesehenen Stellen entlang der Schlauchanordnung zu unterbinden. Außerdem werden unterschiedliche Pole innerhalb des Elektrodenkörpers elektrisch voneinander getrennt. So können die Seile bzw. Filamente der Wendel einzeln isoliert sein oder durch einen axialen Schlauch ineinander geschoben sein.

Der die Wendel umgebende Isolationskörper benötigt zur Erfüllung der vorstehend umrissenen Aufgaben eine Schlauchwand mit einer möglichst großen Wandstärke. Dies führt zu Schlauchanordnungen mit unerwünscht großen Durchmessern. Ferner muss eine Schlauchanordnung, die ausreichend stabil ist, um die radialen Punkt- und Linienlasten ausreichend von der elektrischen Zuleitung abzuhalten, entweder sehr dick oder sehr steif sein, was bezüglich wichtiger Produkteigenschaften, wie Biegbarkeit und kleiner Durchmesser, kontraproduktiv ist.

Herkömmliche Isolationskörper weisen Schlauchanordnungen auf, die nur in begrenztem Maße Zugspannungen aufnehmen können. So ist beobachtet worden, dass es bei der Extraktion eines Elektrodenkatheters wiederholt zu Wendelentdehnungen kommt, da die E-lektrodenkonstruktion um den Elektrodenkörper die erforderlichen Zugspannungen nicht aufnehmen kann. Auch im Hinblick auf die auftretenden Biegespannungen in der Wendel muss ein herkömmlicher Elektrodenkörper eine entsprechend steife Schlauchanordnung aufweisen, um Brüche der Wendel in Folge von Biegebeanspruchungen zu verhindern.

Ausgehend von den geschilderten Problemen des Standes der Technik liegt der Erfindung die Aufgabe zugrunde, eine implantierbare medizinische Schlauchanordnung anzugeben, die bei kompakten Abmessungen der Schlauchwand in ihrem Lastaufnahmeverhalten bezüglich radialer Lasten, Biegebeanspruchungen und Zugbelastungen verbessert ist.

Diese Aufgabe wird erfindungsgemäß dadurch gelöst, dass die Schlauchwand aus mindestens einem wendelförmig gewickelten Profilteil gebildet ist, dessen in Wendel-Umlaufrichtung verlaufender Seitenrand mit dem Seitenrand des benachbarten Abschnitts des Profilteils durch eine Eingriffsmimik verbunden ist. Ferner weist das Profilteil einen elektrischen Leiter auf oder ist elektrisch leitend ausgerüstet.

Die erfindungsgemäße Lösung weist verschiedene Vorteile auf. So wird der Biegeradius der Schlauchanordnung durch die Ausbildung der Eingriffsmimik konstruktiv begrenzt. Der Einlagestrang, der beispielsweise durch Zuleitungen bei der Ausbildung der Schlauchanordnung als Elektrodenkatheter gebildet ist, wird optimal vor schädlichen Biegebeanspruchungen, wie beispielsweise Knicken, geschützt. Außerdem erfolgt ein Schutz vor Torsion, die bei einer Wendel aus der Zugbeanspruchung resultiert, und vor Quetschen. Letzteres passiert zum Beispiel, wenn die Elektrode mit einer Ligatur festgesetzt wird. Üblicherweise wird dafür eine Schutzhülse (Suture Sleeve) verwendet. Bei zu starkem Anziehen der Ligatur kann der herkömmliche Wendelverband zerstört werden. Die Schlauchanordnung kann ferner eine hohe Zugkraft aufnehmen, ohne sich zu dehnen. Dabei werden vom Einlagestrang selbst überhaupt keine Kräfte aufgenommen. Dieser ist also gegen Zugbelastungen optimal geschützt.

Die Schlauchanordnung ist auch sehr widerstandsfähig gegenüber radialen Kräften, wie einem Radialdruck, der bei einem sogenannten Subclavian Crush oder bei Anliegen einer Ligatur auftritt. Eine zusätzliche Isolation des metallischen Einlagestrangs erübrigt sich ebenfalls, da der Einlagestrang durch seine Anordnung am oder im Profilteil ausreichend isoliert ist.

Zusammenfassend können bei der erfindungsgemäßen Schlauchanordnung anderen Materialien und kleinere Dimensionen für den mindestens einen Einlagestrang in der Schlauchwand verwendet werden, da der Einlagestrang keine mechanischen Kräfte mehr aufnehmen muss. So kann bei einem sogenannten Verbunddraht als den Einlagestrang bildende Zuleitung einer als Elektrodenkatheter ausgebildeten Schlauchanordnung die stabilisierende MP35N-Schicht, welche bei herkömmlichen MP35N/Silber-Wendeln jegliche Beanspruchungen aufnimmt, stark reduziert oder es kann sogar darauf verzichtet werden.

In den abhängigen Ansprüchen sind vorteilhafte Weiterbildungen der erfindungsgemäßen Schlauchanordnung angegeben. Was die Erzeugung der elektrisch leitfähigen Struktur in der Schlauchanordnung betrifft, kann mit dem Profilteil mindestens ein metallischer, draht- oder seilartiger Einlagestrang verbunden sein. Alternativ dazu ist es möglich, das Profilteil selbst elektrisch leitfähig auszurüsten, also mit einer leitfähigen, galvanisch oder etwa in einem Siebdruckverfahren aufgebrachten Leitschicht zu versehen oder als elektrisch leitfähiges Profilteil, insbesondere als Metallprofilteil oder als aus elektrisch leitfähigem Kunststoff bestehendes Profilteil, auszubilden. Dann ist dieses Profilteil in vorteilhafter Weise durch eine maßgeschneiderte Isolierung elektrisch abzuschirmen.

Bezüglich der Ausgestaltung des oder der Profilteile insbesondere im Hinblick auf die Eingriffsmimik sind ebenfalls eine Vielzahl von vorteilhaften Varianten möglich. So kann das Profilteil eine im Wesentlichen bandförmige Querschnittskontur aufweisen, wobei an den in Profil-Längsrichtung verlaufenden Seitenrändern dann jeweils eine Eingriffsmimik angeordnet ist. Damit lässt sich eine relativ dünne Schlauchwand realisieren.

Weitere bevorzugte Weiterbildungen betreffen die Ausbildung der Eingriffsmimik, die eine Art Formschlussverbindung bildet. Es können nämlich wechselseitig in Radialrichtung der Schlauchanordnung offene Nuten mit darin unter Axialspiel radial eingreifende Stegvorsprünge vorgesehen sein. Eine Intensivierung des Formschluss-Eingriffs ergibt sich dadurch, dass die genannten Nuten in axialer Richtung der Schlauchanordnung hinterschnitten sein und die Stegvorsprünge dementsprechend in axialer Richtung weisende Fortsätze aufweisen können. Bei dieser Ausbildung ist die Beanspruchbarkeit der Schlauchanordnung in Zugrichtung weiter verbessert, ohne die Biegbarkeit zu beeinträchtigen.

Die Verbindung des Einlagestranges mit dem Profilteil kann ebenfalls auf unterschiedliche bevorzugte Weisen erfolgen. So kann der mindestens eine Einlagestrang direkt in das Profilteil eingebettet sein, was bevorzugtermaßen durch eine Koextrusion des Einlagestranges mit dem Profilteil erreicht werden kann. Alternativ dazu kann der Einlagestrang jeweils in einen im Profilteil ausgebildeten, vorzugsweise in axialer Richtung der Schlauchanordnung offenen Aufnahmekanal eingesetzt sein, in dem der Einlagestrang eingeklebt oder mechanisch gehalten sein kann. Letzteres kann etwa durch Einklipsen oder Einklemmen erfolgen. Bei beiden Varianten ist eine ausreichende Isolierung des Einlagestrangs gewährleistet. Der Aufnahmekanal selbst kann etwa mittig bezogen auf die Breite des Profilteils oder an einem oder beiden Seitenrändern davon verlaufen.

Gemäß einer weiteren bevorzugten Ausführungsform kann das Profilteil mehrteilig aus einem inneren und einem äußeren Profilstrang bestehen, die in axialer Richtung der Schlauchanordnung abwechselnd miteinander eingreifend aneinandergereiht sind. Damit ist es möglich, beispielsweise ein den Einlagestrang führendes Außenteil mit einem klammerartigen Innenteil zu stabilisieren.

Bei der bevorzugten Ausführungsführung der Schlauchanordnung als Elektrodenleitung fungiert der Einlagestrang als elektrisch leitfähige, gewendelte Zuleitung zu einem elektrischen Pol, Sensor oder Aktuator an einem distalen Ende dieser Elektrodenleitung.

Der Werkstoff für das Profilteil kann aus einem oder mehreren der folgenden Materialien ausgewählt sein: PEEK, Polyurethan, Polyamid, Polyimid, PTFE, ETFE, Silikon, Polysulfon, Copolymere aus den vorstehenden Materialien, hochdichtes Polyethylen, Polyethylen, Perfluorethylenpropylen-Copolymer (FEP), oder aus keramischen Werkstoffen, wie A1203, Zr02, Ti02, MgO, ZnO, Aluminiumtitanat (A1203 + Ti02), Bariumtitanat (BaO + Ti02), Siliciumcarbid (SiC), Berylliumoxid (BeO), Aluiniumnitrid (A1N), Hafniumcarbid (HfC), Tantalcarbid (TaC), Titannitrid (TiN), Bornitrid (BN), Borcarbid (B4C), Wolframcarbid (WC), Siliciumnitrid (Si3N4), Metall, elektrisch leitfähiger Kunststoff oder Glas.

Bei Verwendung eines der angegebenen keramischen Werkstoffe, die in der Regel sinterfähig sind, ist produktionstechnisch von Vorteil, das Profilteil vor dem Sintern zu einer Wendel zu wickeln und anschließend den Sinterungsvorgang durchzuführen. Bei einem eingebetteten Einlagestrang wird dieser zusammen mit dem keramischen Werkstoff koextrudiert und anschließend durch den Sintervorgang fest eingebettet. Bei einem beispielsweise eingeklipsten Einlagestrang kann dieser Fügeschritt nach dem Sintern des Profilteils durchgeführt werden.

Für das Material des Einlagestranges ist vorzugsweise ein übliches Seil aus einem medizinischen, leitfähigen Litzenmaterial oder ein gewendelter Draht oder ein Profil aus einem leitfähigen Kunststoff denkbar.

Die Struktur der Eingriffsmimik, also beispielsweise die Art des Formschlusses der ineinandergreifenden Komponenten der Profilteile, kann über die Länge der Schlauchanordnung variiert werden, so dass diese an unterschiedlichen Axialpositionen variable Biegeeigenschaften aufweist. So können unterschiedliche Biegeradien an unterschiedlichen Längspositionen der Schlauchanordnung realisiert werden.

Weitere Merkmale, Einzelheiten und Vorteile der Erfindung ergeben sich aus der nachfolgenden Beschreibung, in der verschiedene Ausführungsbeispiele anhand der beigefügten Zeichnungen näher erläutert werden. Es zeigen:
- Fig. 1 und 2: perspektivische, teilweise weggeschnittene Ansichten einer Schlauchanordnung in einer ersten Ausführungsform,
- Fig. 3: eine perspektivische Teildarstellung des Profilteils mit einer elektrischen Zuleitung,
- Fig. 4: eine perspektivische, teilweise weggeschnittene Ansicht einer Schlauchanordnung in einer zweiten Ausführungsform,
- Fig. 5: eine perspektivische Teildarstellung des Profilteils gemäß Fig. 4 mit zwei darin eingesetzten Zuleitungen,
- Fig. 6 bis 8: perspektivische, teilweise weggeschnittene Darstellungen einer Schlauchanordnung in einer dritten Ausführungsform,
- Fig. 9: eine perspektivische Teilansicht des Profilteils gemäß Fig. 6 bis 8 mit einer darin eingebetteten Zuleitung,
- Fig. 10 bis 12: perspektivische, teilweise weggeschnittene Ansichten einer Schlauchanordnung in einer vierten Ausführungsform,
- Fig. 13: eine perspektivische Teildarstellung des Profilteils gemäß Fig. 10 bis 12 mit vier eingebetteten Zuleitungen,
- Fig. 14 und 15: perspektivische, teilweise weggeschnittene Darstellungen einer Schlauchanordnung in einer fünften Ausführungsform,
- Fig. 16: eine perspektivische Teildarstellung des Profilteils gemäß Fig. 14 und 15 mit einer eingebetteten Zuleitung,
- Fig. 17: eine perspektivische, teilweise weggeschnittene Darstellung einer Schlauchanordnung in einer sechsten Ausführungsform,
- Fig. 18: eine perspektivische Teilansicht eines aus zwei Komponenten zusammengesetzten Profilteils gemäß Fig. 17,
- Fig. 19: eine perspektivische, teilweise weggeschnittene Darstellung einer Schlauchanordnung in einer siebten Ausführungsform,
- Fig. 20: eine perspektivische Teilansicht eines aus zwei Komponenten zusammengesetzten Profilteils gemäß Fig. 19,
- Fig. 21: eine perspektivische, teilweise weggeschnittene Darstellung einer Schlauchanordnung in einer achten Ausführungsform mit vier eingesetzten Zuleitungen,
- Fig. 22: perspektivische, teilweise weggeschnittene Darstellung einer Schlauchanordnung in einer neunten Ausführungsform, und
- Fig. 23: eine perspektivische Teildarstellung des dabei verwendeten Profilteils.

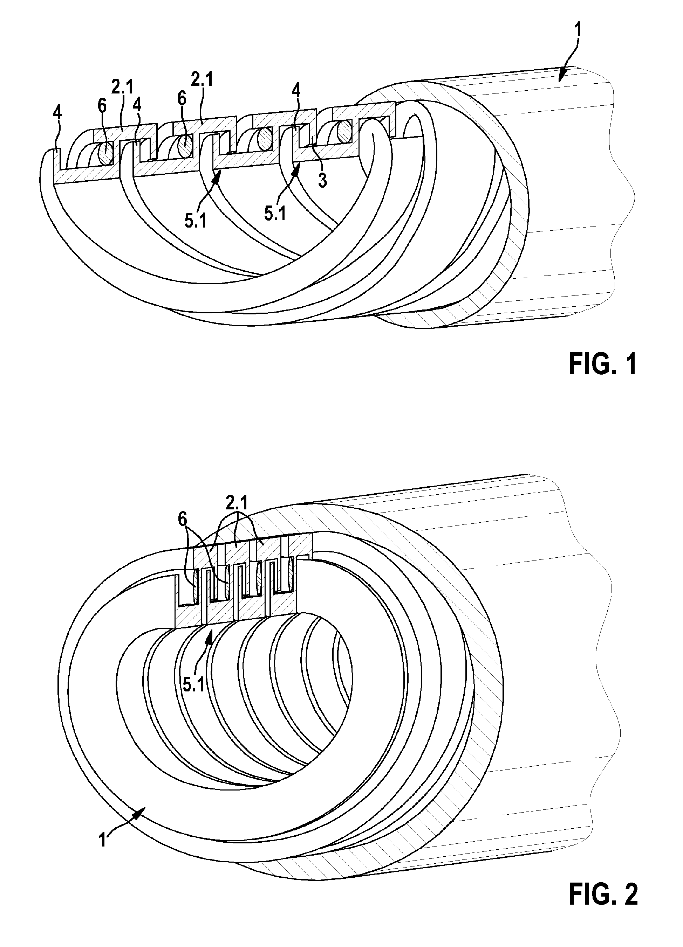

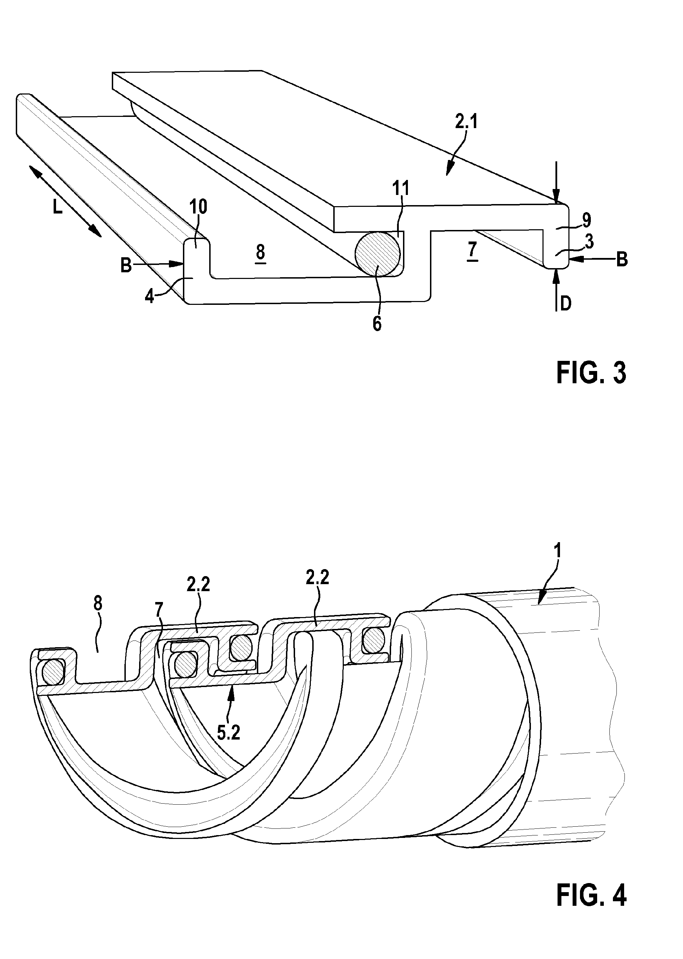

Alle Ausführungsbeispiele der gezeigten Schlauchanordnungen beruhen auf einem übereinstimmenden Bauprinzip, das anhand der Fig. 1 bis 3 erörtert werden soll. So wird für den Aufbau einer langgestreckten Schlauchanordnung mit einer Schlauchwand 1 ein Profilteil 2.1 verwendet, das - wie Fig. 3 deutlich macht - eine bandförmige Kontur aufweist. Darunter ist zu verstehen, dass die Breitendimension B quer zur Profillängsrichtung L ein Vielfaches höher ist als die die Schlauchwand 1 bildende Dicke D des Profilteils 2.1. Wie aus Fig. 1 und 2 deutlich wird, wird das Profilteil 2.1 wendelförmig, vorzugsweise auf Block gewickelt, so dass sich eine geschlossene Schlauchwand 1 ergibt. Für den Zusammenhalt der Windungen des Profilteils 2.1 ist dieses an seinen beiden Seitenrändern 3, 4 mit einer Eingriffsmimik 5.1 versehen, mit deren Hilfe die benachbarten Abschnitte der einzelnen Wickelungen des Profilteils 2.1 in noch näher zu erörternder Weise verbunden sind.

Mit dem Profilteil 2.1 ist ein metallischer Einlagestrang in Form eines Wendeldrahtes 6 verbunden, der für die elektrische Kontaktierung beispielsweise einer hier nicht dargestellten Elektrode eines kardiologischen Elektrodenkatheters vorgesehen sein kann.

Bei der in Fig. 1 bis 3 gezeigten Ausführungsform des Profilteils 2.1 ist die Eingriffsmimik 5.1 einerseits durch eine in Radialrichtung R offene Nut 7, 8 entlang der beiden Seitenränder 3, 4 gebildet, die von außen entlang der Seitenränder 3, 4 führenden Stegvorsprünge 9, 10 außenseitig begrenzt werden. Wie insbesondere aus Fig. 1 deutlich wird, greifen die jeweiligen Stegvorsprünge 9 und 10 der beiden benachbarten Abschnitte des Profilteils 2.1 jeweils in die Nuten 7, 8 unter Axialspiel ein, so dass die Integrität der Schlauchwand 1 über die Länge der Schlauchanordnung gewährleistet, gleichzeitig aber eine flexible Biegung der Schlauchwand durch Verschieben der jeweiligen Stegvorsprünge 9, 10 in den Nuten 7, 8 ermöglicht wird.

Der Wendeldraht 6 ist beim Profilteil 2.1 in einen mittig verlaufenden, zur Seite hin offenen Aufnahmekanal 11 eingeklipst, eingeklebt oder mechanisch geklemmt.

Produktionstechnisch wird das Profilteil 2.1, das beispielsweise aus PEEK besteht, zuerst extrudiert und dann der Wendeldraht 6 in den Aufnahmekanal 11 eingesetzt. Anschließend werden im gezeigten Beispiel vier Profilteile 2.1 aneinandergesetzt und gewickelt, so dass ein Vierfach-Strang von Wendeldrähten 6 entlang der Schlauchwand 1 geführt ist.

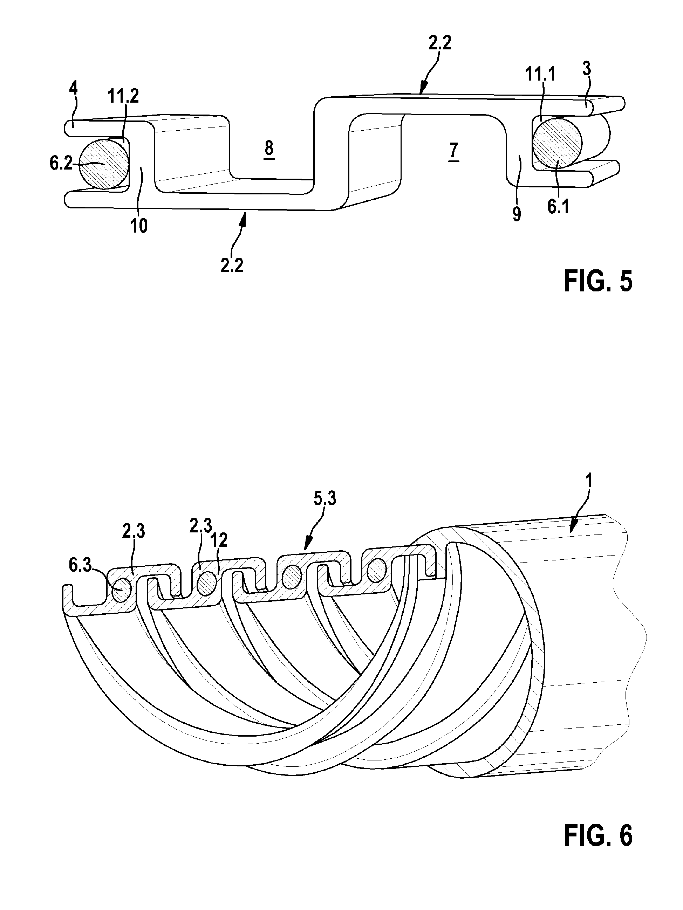

Die in Fig. 4 und 5 gezeigte Ausführungsform einer Schlauchanordnung basiert auf einem Profilteil 2.2, bei dem wiederum eine Eingriffsmimik 5.2 mit Nuten 7, 8 und Stegvorsprüngen 9, 10 vorgesehen sind. Im Unterschied zum Ausführungsbeispiel gemäß den Fig. 1 bis 3 sind hier in einem Profiteil 2.2 zwei Wendeldrähte 6.1, 6.2 vorgesehen, deren Aufnahmekanäle 11.1, 11.2 jeweils entlang der Seitenränder 3, 4 vorgesehen sind. Auch hier erfolgt die Befestigung der Wendeldrähte 6.1, 6.2 in den Aufnahmekanälen 11.1, 11.2 über Einklipsen, Kleben oder mechanische Klemmung.

Wie aus Fig. 4 deutlich wird, liegen die Stegvorsprünge 9, 10 mit den daran ausgebildeten Aufnahmekanälen 11.1, 11.2 in den jeweiligen Nuten 7, 8, wodurch ein wechselseitiger Eingriff mit entsprechend stabiler Verbindung zwischen den einzelnen Windungen des Profilteils 2.2 stattfindet.

Die Herstellungsweise erfolgt analog dem Ausführungsbeispiel gemäß Fig. 1 bis 3, wobei wiederum mehrere Profilteile 2.2 nebeneinander zu einem Mehrfachstrang gewickelt werden können.

Das in den Fig. 6 bis 9 gezeigte Ausführungsbeispiel einer Schlauchanordnung basiert auf einem Profilteil 2.3, das analog den vorherigen Ausführungsbeispielen eine Eingriffsmimik 5.3 mit Nuten 7, 8 und Stegvorsprüngen 9, 10 aufweist. Insoweit besteht in der Wicklung und der Eingriffsverbindung zwischen den einzelnen Wicklungen des Profilteils 2.3 kein signifikanter Unterschied zu den vorherigen Ausführungsbeispielen.

Abweichend ist die Unterbringung des Wendeldrahtes 6.3, der hier in einem zentralen Kernabschnitt 12 des Profilteils 2.3 vollständig eingebettet untergebracht ist. Zu diesem Zweck wird der Wendeldraht 6.3 mit dem Profilteil 2.3 koextrudiert. Anschließend werden wieder mehrere Profilteile 2.3 durch die in den Fig. 6 bis 8 gezeigte Eingriffsmimik 5.3 aneinandergehängt und vorzugsweise auf Block gewickelt, so dass eine durchgehende Schlauchwand 1 mit vollständig eingebetteten Wendeldrähten 6.3 realisiert wird. Wie in diesen Zeichnungen nicht näher dargestellt ist, können bei Verwendung mehrerer auf sich gewickelter Profilteile 2.3 Blindprofilteile zwischen den die Wendeldrähte 6.3 aufweisenden Profilteilen 2.3 angeordnet sein.

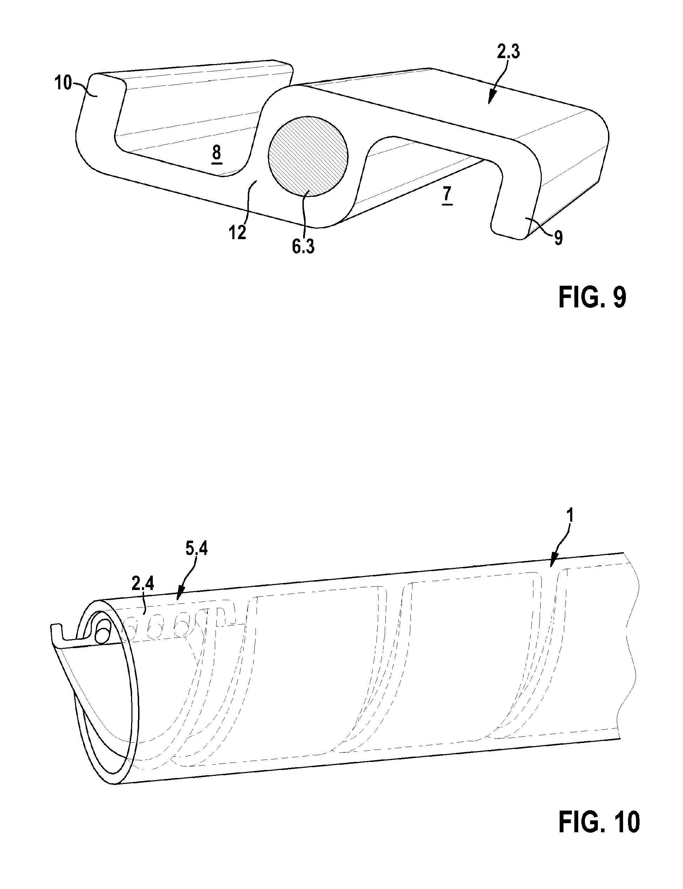

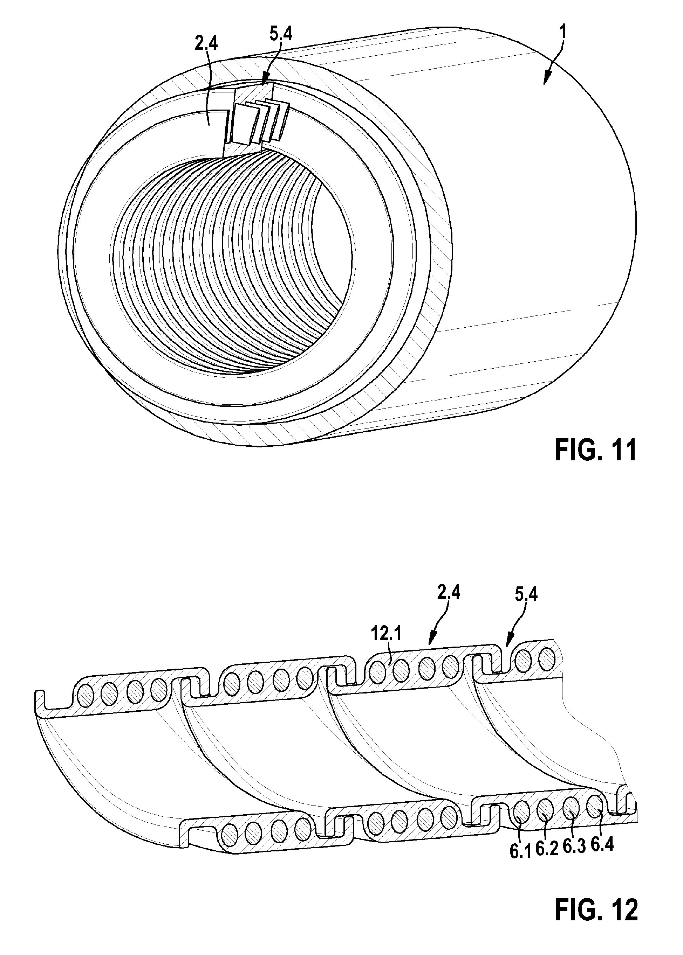

Bei der in den Fig. 10 bis 13 gezeigten Ausführungsform werden vier Wendeldrähte 6.4 parallel nebeneinanderliegend in einen verbreiterten Kernabschnitt 12.1 einextrudiert. Anschließend wird dieses Profilteil 2.4 mit der gleichen Eingriffsmimik 5.4 vorzugsweise auf Block gewickelt und fixiert, wie sie in den Ausführungsbeispielen gemäß Fig. 1 bis 3 und 6 bis 9 verwendet wird. Es greifen also wieder die Stegvorsprünge 9, 10 wechselseitig in die Nuten 7, 8 ein, um so eine zugsichere, aber biegsame Verbindung zwischen den einzelnen Wicklungen des Profilteils 2.4 zu gewährleisten.

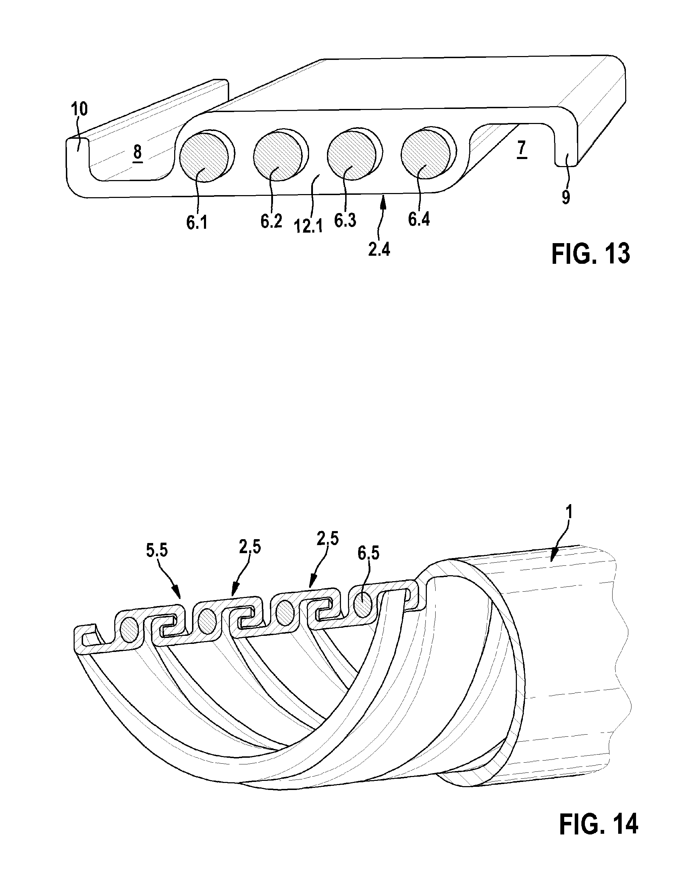

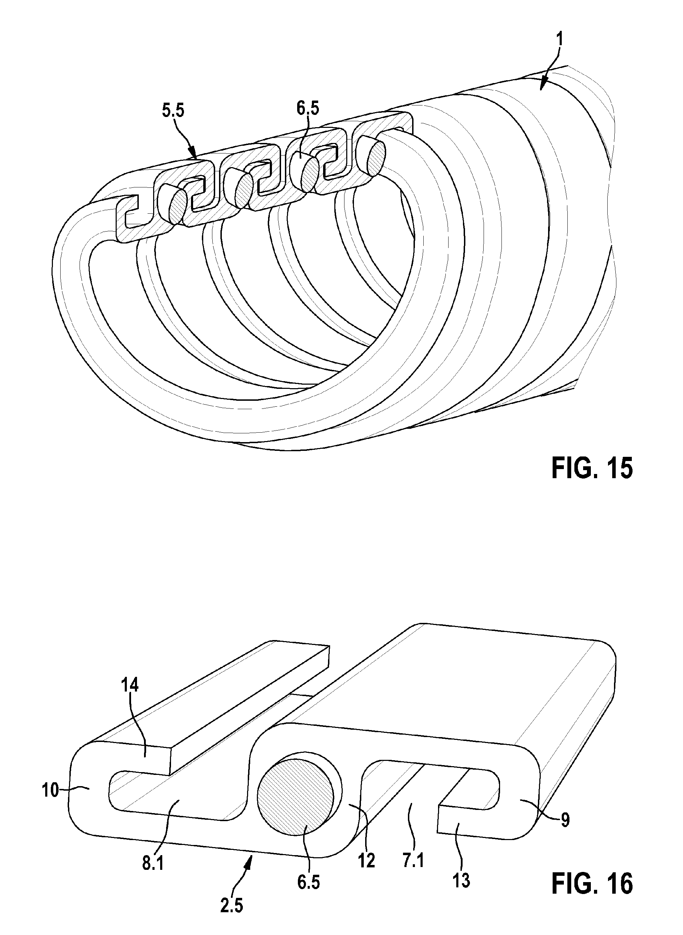

Die in den Fig. 14 bis 16 gezeigte Ausführungsform leitet sich von der Ausführungsform gemäß den Fig. 6 bis 9 ab, d. h., dass der Wendeldraht 6.5 wiederum zentral in das Profilteil 2.5 eingebettet ist. Im Unterschied zur vorgenannten Ausführungsform gemäß den Fig. 6 bis 9 ist die Eingriffsmimik 5.5 zusätzlich verstärkt, indem die Nuten 7.1, 8.1 in axialer Richtung der Schlauchanordnung hinterschnitten sind. Dies wird durch dementsprechend in axialer Richtung weisende Fortsätze 13, 14 an den Stegvorsprüngen 9, 10 realisiert, so dass - wie Fig. 14 verdeutlicht - die benachbarten Wicklungen des hier wieder vierfach nebeneinanderliegenden Profilteils 2.5 sich miteinander verkrallen. Dies stellt eine ganz besonders stabile, gleichzeitig aber biegsame Verbindungsweise dar. Der Wendeldraht 6.5 ist in den Kernabschnitt 12 einextrudiert.

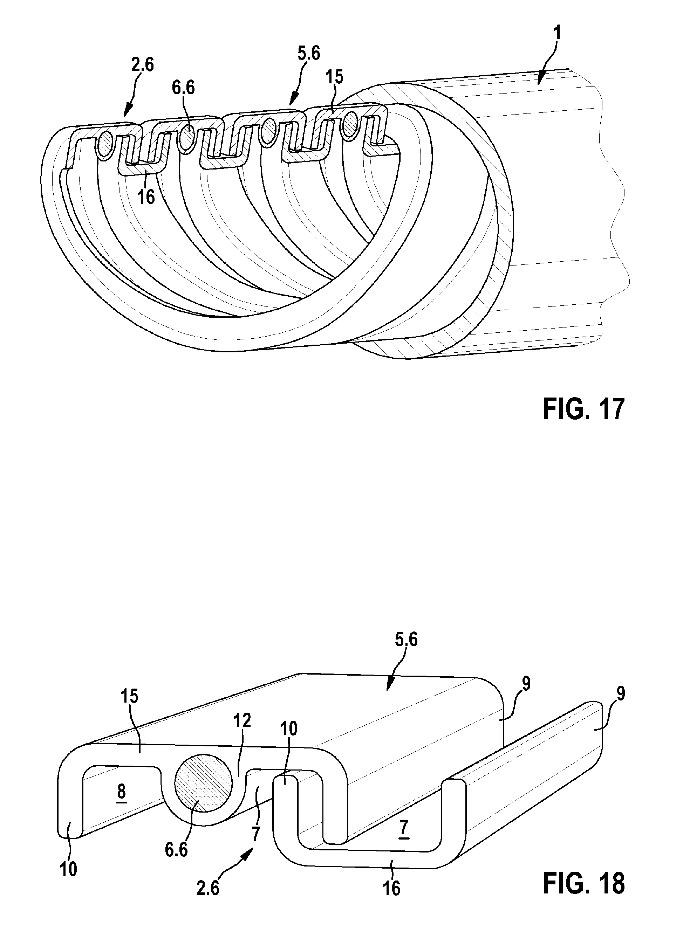

In Fig. 17 und 18 ist eine Schlauchanordnung gezeigt, bei der das Profilteil 2.6 aus zwei Komponenten zusammengesetzt ist, nämlich einem außenliegenden Profilstrang 15 und einem innenliegenden Profilstrang 16. Beiden Profilstränge 15, 16 sind im Querschnitt etwa flach-U-förmig ausgebildet. Die U-Schenkel bilden damit die Stegvorsprünge 9, 10 der Eingriffsmimik 5.6 des Profilteils 2.6, die wechselseitig in den von den U-Schenkeln und der U-Basis der Profilstränge 15, 16 umgebenden Innenraum als Nuten 7, 8 eingreifen. Damit können wechselseitig äußere und innere Profilstränge 15, 16 ineinandergreifen und vorzugsweise "auf Block" gewickelt werden. Der äußere Profilstrang 15 trägt dabei in einem Kernabschnitt 12 wiederum einen mit einextrudierten Wendeldraht 6.6.

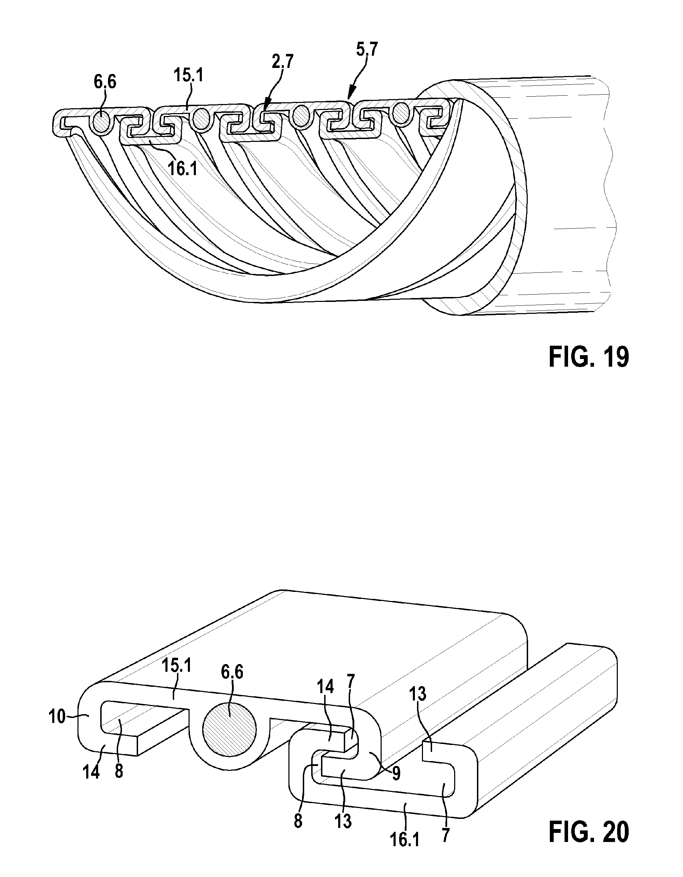

Die Ausführungsform gemäß den Fig. 19 und 20 unterscheidet sich von der gemäß Fig. 17 und 18 darin, dass die beiden Profilstränge 15.1, 16.1 mit hinterschnittenen Nuten 7, 8 durch die klammerartig einwärts verlaufenden Fortsätze 13, 14 eine an den Stegen 9, 10 hinterschnittene Eingriffsmimik 5.7 aufweisen, wie dies näher anhand der Fig. 14 bis 16 bereits erläutert wurde.

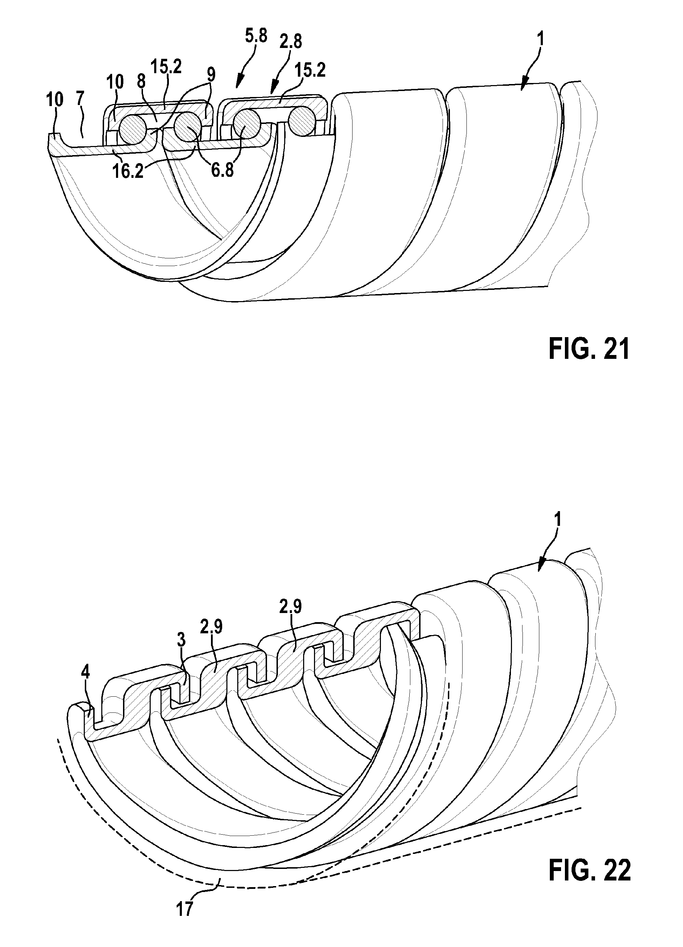

Bei der in Fig. 21 gezeigten Ausführungsform wird das Profilteil 2.8 wieder durch einen äußeren Profilstrang 15.2 und einen inneren Profilstrang 16.2 gebildet, die wechselweise versetzt zueinander mit ihren Stegvorsprüngen 9, 10 in die entsprechende Nut 7, 8 zur Bildung der Eingriffsmimik 5.8 eingreifen. Die Besonderheit an dieser Ausführungsform ist die Tatsache, dass die Wendeldrähte 6.8 Teil der Eingriffsmimik 5.8 sind, indem die Stegvorsprünge 9, 10 auch über die jeweiligen Wendeldrähte 6.8 übergreifen.

Produktionstechnisch werden die Wendeldrähte zuerst mit einer größeren Steigung als die Wicklungen des Profilteils 2.8 gewickelt und dann unter den äußeren Profilstrang 15.2 bzw. über den inneren Profilstrang 16.2 geschoben. Anschließend werden die Profilstränge 15.2, 16.2 miteinander verbunden, so dass die in Fig. 21 gezeigte Konfiguration entsteht. In dieser sind die einzelnen Profilstränge 15.2, 16.2 in axialer Richtung aneinander gehalten, flexibel biegbar und die Wendeldrähte 6.8 zuverlässig voneinander isoliert.

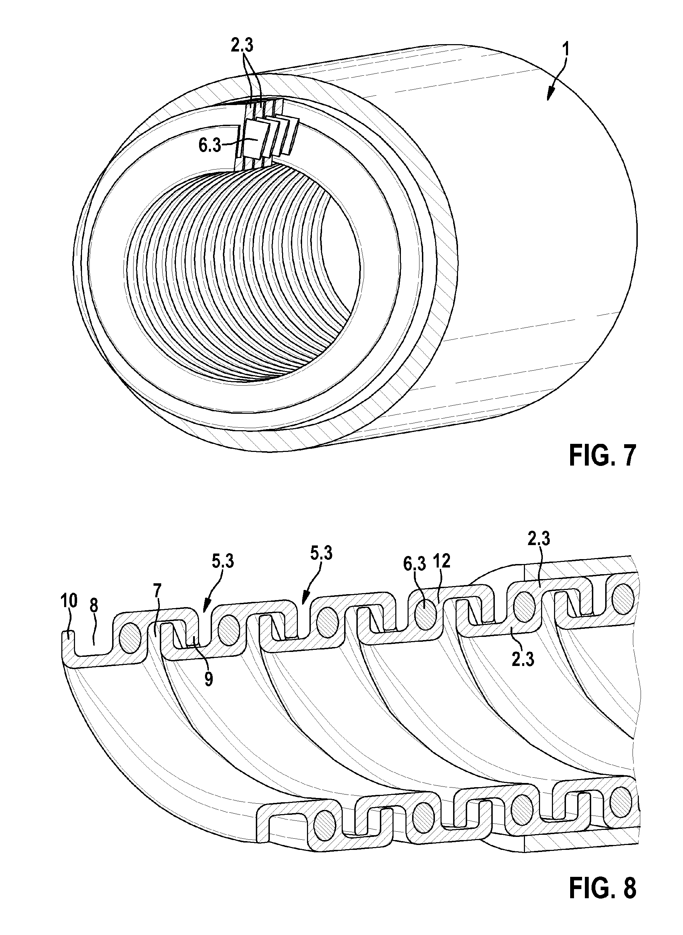

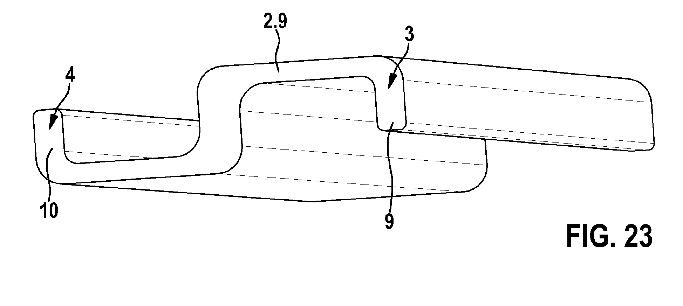

Bei der in den Fig. 22 und 23 gezeigten Ausführungsform wird statt des Kunststoffprofilteils ein Metallprofilteil 2.9 verwendet, das ausgehend von üblichen Flachbandwendeln weiterentwickelt ist und demgemäß die erfindungsgemäße Kontur und den gegenseitigen Eingriff der Profilteile analog beispielsweise Fig. 8 und 9 aufweist. Um die aus diesem Profilteil 2.9 gebildete Schlauchwand 1 ist eine nur gestrichelt angedeutete Isolierung 17 gelegt. Die so entstehende Wendel dient als Innen- oder Außenwendel und macht die Elektrode stabil. Das Profilteil 2.9 kann auch durchgehend aus elektrisch leitfähigem Kunststoff bestehen.

## Patentansprüche

1. Implantierbare medizinische Schlauchanordnung, umfassend eine auf Biege-, Zug- und radiale Drucklasten beanspruchbare Schlauchwand (1),
**dadurch gekennzeichnet, dass**
- die Schlauchwand (1) aus mindestens einem wendelförmig gewickelten Profilteil (2) gebildet ist, dessen in Wendel-Umlaufrichtung verlaufender Seitenrand (3) mit dem Seitenrand (4) des benachbarten Abschnitts des Profilteils (2) durch eine Eingriffsmimik (5) verbunden ist, und
- das Profilteil (2) einen elektrischen Leiter aufweist oder elektrisch leitend ausgerüstet ist.

2. Schlauchanordnung nach Anspruch 1, **dadurch gekennzeichnet, dass** das Profilteil (2) auf Block gewickelt ist.

3. Schlauchanordnung nach Anspruch 1 oder 2, **dadurch gekennzeichnet, dass** mit dem Profilteil (2) mindestens ein metallischer, draht- oder seilartiger Einlagestrang (6) verbunden ist.

4. Schlauchanordnung nach Anspruch 1 oder 2, **dadurch gekennzeichnet, dass** das Profilteil (2) als elektrisch leitfähiges Profilteil, insbesondere als Metallprofilteil (2.9) oder als aus elektrisch leitfähigem Kunststoff bestehendes Profilteil, ausgebildet ist.

5. Schlauchanordnung nach Anspruch 4, **dadurch gekennzeichnet, dass** das Metallprofilteil (2.9) oder aus elektrisch leitfähigem Kunststoff bestehende Profilteil mit einer Isolation (17) versehen ist.

6. Schlauchanordnung nach einem der vorgenannten Ansprüche, **dadurch gekennzeichnet, dass** bei Verwendung mehrerer auf sich gewickelter Profilteile (2) Blindprofilteile zwischen den elektrisch leitend ausgerüsteten oder einen elektrischen Leiter aufweisenden Profilteilen (2) angeordnet sind.

7. Schlauchanordnung nach einem der vorgenannten Ansprüche, **dadurch gekennzeichnet, dass** das Profilteil (2) eine bandförmige Kontur aufweist, an deren im Profil-Längsrichtung (L) verlaufenden Seitenränder (3, 4) jeweils eine Eingriffsmimik (5) angeordnet ist.

8. Schlauchanordnung nach einem der vorgenannten Ansprüche, **dadurch gekennzeichnet, dass** die Eingriffsmimik (5) durch wechselseitig in Radialrichtung (R) der Schlauchanordnung offene Nuten (7, 8) mit darin unter Axialspiel radial eingreifenden Stegvorsprüngen (9, 10) gebildet ist.

9. Schlauchanordnung nach Anspruch 8, **dadurch gekennzeichnet, dass** die Nuten (7, 8) in axialer Richtung der Schlauchanordnung hinterschnitten sind und die Stegvorsprünge (9, 10) dementsprechend in axialer Richtung weisende Fortsätze (13, 14) aufweisen.

10. Schlauchanordnung nach einem der Ansprüche 1 bis 9, **dadurch gekennzeichnet, dass** der mindestens eine Einlagestrang (6) jeweils in einem im Profilteil (2) ausgebildeten, vorzugsweise in axialer Richtung der Schlauchanordnung offenen Auf-nahmekanal (11) eingesetzt ist.

11. Schlauchanordnung nach Anspruch 10, **dadurch gekennzeichnet, dass** der Einlagestrang (6) in den Aufnahmekanal (11) eingeklebt oder mechanisch gehalten, vorzugsweise eingeklipst oder eingeklemmt ist.

12. Schlauchanordnung nach einem der vorgenannten Ansprüche, **dadurch gekennzeichnet, dass** das Profilteil (2) aus einem inneren und einem äußeren Profilstrang (15, 16) besteht, die in axialer Richtung der Schlauchanordnung abwechselnd miteinander eingreifend aneinandergereiht sind.

13. Schlauchanordnung nach einem der vorgenannten Ansprüche in Form einer Elektrodenleitung für die Stimulation und/oder Wahrnehmung von Muskel- und/oder Nervenaktivitäten und/oder zur Übertragung von Daten oder Ansteuerung von Sensoren oder Aktuatoren, **dadurch gekennzeichnet, dass** der Einlagestrang (6) als elektrisch leitfähige, gewendelte Zuleitung zu einem elektrischen Pol, Sensor oder Aktuator am distalen Ende der Elektrodenleitung ausgebildet ist.

14. Schlauchanordnung nach einem der vorgenannten Ansprüche, **dadurch gekennzeichnet, dass** der Einlagestrang (6) aus einem Seil oder einem gewendelten Draht besteht.

15. Schlauchanordnung nach einem der vorgenannten Ansprüche, **dadurch gekennzeichnet, dass** die Eingriffsmimik (5) über die Länge der Schlauchanordnung derart variiert, dass die Schlauchanordnung an unterschiedlichen Axialpositionen variable Biegeeigenschaften aufweist.
